# EUROPEAN PATENT APPLICATION

(11) **EP 1 897 521 A1**
(43) Date of publication of application: **12.03.2008**
(21) Application number: 06120098.6
(22) Date of filing: 05.09.2006
(51) Int. Cl.: A61F 13/495, A61F 13/512

(54) **Absorbent articles with elasticated topsheet with reduced leakage**

(71) Applicant: The Procter and Gamble Company, Cincinnati, Ohio 45202 (US)
(72) Inventor: Wciorka, Maja, 65824, Schwalbach (DE); Ponomarenko, Ekaterina Anatolyevna, 65812, Bad Soden (DE)
(74) Representative: McGregor, Judit Ester

(57) **Abstract**

The present invention relates to an absorbent article that has a topsheet (30) with elastic components (40) and one or more openings (31) with longitudinal side edges (32) and a connecting edge (33 a, b) in the back region (3), whereby the angel between a longitudinal side edge (32) and connecting edge (33) (angle α) is from 80° to 105°, and preferably whereby the ratio of the length L to L", as defined herein, is 0.55 or less, to reduce the risk of the presence of transverse wrinkles and subsequent leakage.

## Description

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

### FIELD OF THE INVENTION

This invention is directed to an absorbent article comprising an elastic topsheet made of one or more sheets with one or more elastic components and having one or more large openings to receive feces into a void space under the topsheet, said topsheet having in the back region minimal regions where transverse wrinkles may occur, to reduce or eliminate the subsequent risk of leakage of feces through such wrinkles.

### BACKGROUND OF THE INVENTION

In recent years, many diapers have been suggested to help isolating feces and storing it, to reduce the amount of feces that sticks to the skin of the user. For example, diapers with a topsheet with one or more openings, through which the feces can pass to a void space between the topsheet and the absorbent core, have been developed. The fecal material is then stored underneath this topsheet, away from the skin. For example EP 1417947-A and co-pending European application 01117669.0 describe such diapers with a topsheet with a large slit opening with double Y or X-shaped elastics, which serve the keep the topsheet's opening close to the anus and genitals.

The inventors have now found that with many of the proposed topsheets or cuffs of the prior art, some feces leakage may still occur, especially when the feces is runny. The inventors have surprisingly found that when the topsheet comprises in the back region elastics that are curved or angled, such leakage may be stronger and that such leakage may occur when significant transverse wrinkles are present. The inventors found that by forming the topsheet with the elastics and the opening such that (in the back region) only a minimum area is obtained were transverse wrinkles may occur, or by forming the back region of the topsheet such that no substantive transverse wrinkles occur, the risk of leakage can be significantly reduced or eliminated and a more effective feces isolation can be achieved.

### SUMMARY OF THE INVENTION

The invention relates to absorbent article (1) having a backsheet (10), an absorbent core (20) and a topsheet (30) with one or more opening (s) (31) to receive fecal material, each having a longitudinal direction and axis (Y), and a transverse direction and axis (X), said topsheet (30), made from one or more sheet material(s), and having a front region (2) and a back region (3), whereby at least one opening (31) or part thereof is present in at least the back region (3) of the topsheet (30), and whereby the article (1) comprises in relaxed state a void space between said absorbent core (20) and said topsheet (30), whereby in 100% stretched state:
a) said topsheet comprising at least one elastic component(40 a, b), whereby each elastic component (40 a, b) comprises a longitudinally extending portion and optionally a curvature, with a curvature point A, or an angle with an angle point A, present in the back region (3) of the topsheet (30);
b) said opening (31) or part thereof (3) having two substantially longitudinally extending side edges (32 a, b) and at least substantially parallel, adjacent to and in close proximity to each of said longitudinal edges or part thereof, one or more of said longitudinally extending portions of said elastic component(s) (40 a, b);
c) and said opening (31) having at least one connecting edge (33 a, b), connecting the longitudinal edges (32 a, b) in the back region (3);
whereby each connecting edge (33, a, b) connects to a longitudinal side edge (32 a, b) in a point D (with angle α which is from 80° to 105°, preferably from 90° or 95° to 100°).

The invention also relates to a process for making an absorbent article (1) having a backsheet (10), an absorbent core (20) and a topsheet (30) with at least one opening (31) to receive fecal material, each having a longitudinal direction and axis (Y), and a transverse direction and axis (X), said topsheet (30) being made from one or more sheet material(s), and having a front region (2) and a back region (3), whereby at least one opening (31) or part thereof is present in at least the back region (3) of the topsheet (30), comprising the steps of;
a) obtaining a topsheet material with a longitudinal direction (Y) and a transverse direction (X) a back region (3) and a front region (2) and transverse end edges (50 a, b);
b) forming a central longitudinal slit (51) with slit edges through at least part of the back region (3) of the topsheet material in longitudinal direction Y, but not through the transverse end edges (50a, b) of the sheet material and forming a two-dimensional opening (53), e.g. cut-out, in the back region (3) of the sheet, said opening (53) comprising a front connecting edge (33 a, b), and side edges that are connected to one another; and
c) prior, simultaneous with or subsequent to steps b) attaching one or more elastic components (40 a, b) in partially or completely stretched state to said sheet material with each an angle or curvature, having an angle point A or a curvature point A, in at least the back region (3) of said topsheet material, and said elastic components (40 a, b) each comprising a longitudinally extending portion that is substantially parallel to and in close proximity to one of the longitudinal edges or part thereof of said slit (51);
whereby the angle α (in point D) between said front connecting edge (33 a, b) and a side edge of said opening (53) is from 80° to 105°.

The invention also relates to articles obtainable by the process claimed and described herein.
Preferably, the absorbent articles has point C and B and D as defined herein and the ratio of the length L, from D to C to the length L", from C to B is 0.55 or less, preferably 0.50 or less, or preferably 0. 45 or less; and optionally more than 0.10, preferably more than 0.20, or preferably more than 0.30. Point A is typically closer to the transverse axis of the topsheet than point D.
Preferably, the points A, B, C and D are all comprised by the edges of the opening or cut-out (53).

The invention also relates to an absorbent article (1) having a backsheet (10), an absorbent core (20) and a topsheet (30) with at least one opening (31) to receive fecal material, said topsheet (30) having a front region (2) and a back region (3), and having a longitudinal direction and axis (Y) and a transverse direction and axis X, the article (1) comprises a void space between said absorbent core (20) and said topsheet (30), whereby in 75% stretched state :
a) the topsheet (30) is made from one ore more sheet material(s) and comprises at least one elastic component (40 a, b),
b) each elastic component (40 a, b) is at least present along at least part of the longitudinal side edges (32 a, b) of the opening(s) (31), each elastic component (40 a, b) being attached to said topsheet with an angle or curvature, having an angle or curvature point A, in at least said back region (3), and each extending with an extending portion (43) towards the transverse edge and towards the longitudinal edge of the topsheet (30);
d) said opening (31) having a connecting edge (33 a, b), connecting the longitudinal edges (32 a, b) in said back region (3) in points D and each edge having a centre point C;
c) said topsheet (30) having in the back region (3) one ore more triangular portions N, each defined by: i) a portion of the connecting edge (33 a or b) from point D to C; ii) the closest elastic component extending portion (43), closest to said portion from point C to D; and iii) a line connecting i) and ii);
d) said portion(s) N being substantially free of transversely extending wrinkles, having no transversely extending wrinkles of a height of more than 3 mm and any transverse wrinkles present having an average transverse wrinkle height of less than 2 mm.

The absorbent article is preferably a diaper, including pull-on or training pants and/ or adult incontinence articles.

### BRIEF DESCRIPTION OF THE FIGURES

Figure 1 shows a perspective view of a preferred absorbent article (1) of the present invention.

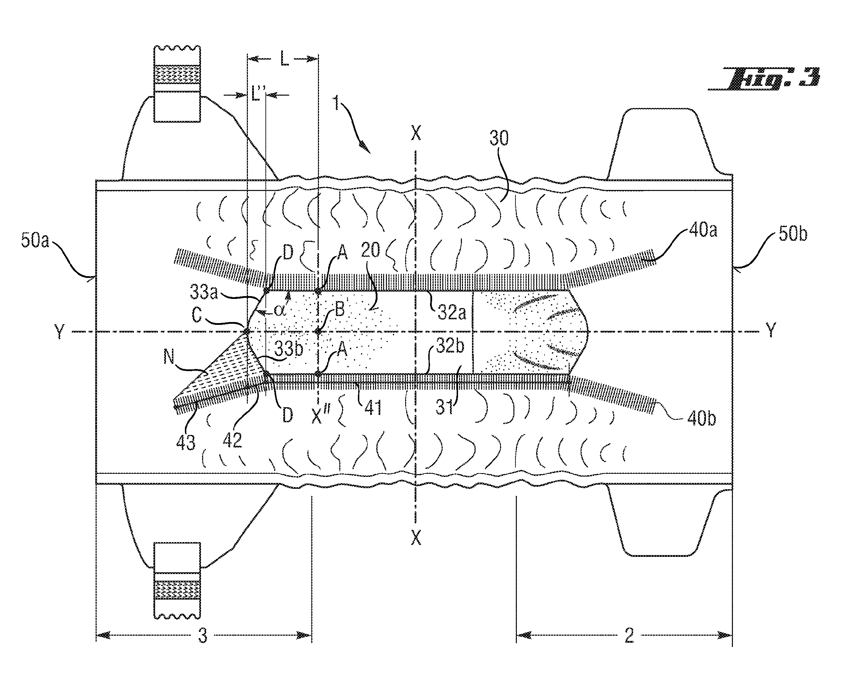

Figure 2 shows a top view of an absorbent article of the prior art.
Figure 3 shows a top view of the preferred absorbent article (1) of Figure 1.
Figures 4 shows a section view of the back region (3) of the article (1) of Figure 1.
Figure 5 shows how a preferred opening (31) may be formed in the sheet material useful as topsheet (30) herein, by cutting out a "diamond-shaped" opening part (53).

### DETAILED DESCRIPTION OF THE INVENTION

As used herein, the following terms have the following meanings.
As used herein, 'absorbent article' means any article that can absorb body fluids and is suitable to be placed in close to or against the genitals of the user, including in particular and adult or infant diaper and so-called training or pull-up pants.
As used herein 'front region' and 'back region' refer to the two regions, which are in use, respectively, closest to the front of the wearer and the back of the wearer, each having half the longitudinal length of the article or topsheet (30).
As used herein, the term 'void space' is a cavity in the article present in at least the relaxed state, which serves to accept and contain bodily exudates such as fecal material, typically being at least 5 cm³ in relaxed state.
As used herein, 'longitudinal' is the direction running substantially parallel to the maximum linear dimension of the component, typically to the longitudinal axis of the article, and includes directions within 30° of this parallel, unless otherwise stated.
The 'transverse' direction is orthogonal to the longitudinal direction and in the same plan of the majority of the article and the longitudinal axis and includes directions within 30° of the orthogonal, unless otherwise specified.
'In close proximity' when used herein with respect to the elastic components and the edges of the opening (31) means that the average distance between the components is at the most 3 mm.
'Adjacent' when used herein with respect to the elastic components and opening's edges means that two adjacent points, parts or areas of two component are present on the same exact transverse line or exact longitudinal line.
As used herein 'relaxed' or 'relaxed state' means the state that no forces are applied to the article or component thereof (other than naturally occurring forces such as gravity), when the article is laid on a horizontal surface, such that the transverse front and back edge are flat on the horizontal surface and the transverse centre line or axis is on the horizontal surface.
As used herein, 'elasticated' means typically, that the component consists of or comprises elastic material, which is elastic in at least one direction. 'Non-elasticated' when used herein means that the component does not comprise any elastic material.
As used herein, 'opening (31) in the topsheet' means an area completely circumscribed by the topsheet or sheet components thereof, but where no topsheet material is present, and which is large enough to receive fecal material, typically being at least 2 cm long or wide, or having a surface area of at least 2 cm².
The "curvature point A" as used herein is the point on the elastic component that is exactly in the middle of the curvature of the elastic component (when the elastic is attached with a curvature). It should be noted that the curvature or angle of the elastic component when the elastic component is attached may be different to the curvature or angel when the elastic component is in the article as in use, but the angle or curvature point A stay in the same place.
All dimensions and the definition of the components of the absorbent article as used herein, such as L, L", angles, are for such an article in 100% stretched state, unless otherwise stated, and except for the definition of N and the wrinkles thereof, which is determined in 75% stretched state (more accurately representing the "in use" state). The 75% stretched state can be obtained by fully stretching the absorbent article and determining its fully stretched state and then relaxing the article partially to the 75% stretched state.

The topsheet (30) of the present invention comprises at least one opening (31) or part thereof in the back region (3), and said opening (31) or part thereof comprises two opposing substantially longitudinal side edges (32 a, b) connected to one another with one or more connecting edges (33 a, b) that connect to each side edge (32) in a point D with an angle α which is from 80° to 105°, preferably from 90° or 95° to 100°.

Preferred may be that the longitudinal side edges (32 a, b) are parallel to the longitudinal axis Y of the topsheet and the article. A preferred connecting edge (33 a, b) has two or more portions (33 a, and 33 b) connected to one another with an angle or curvature. For example, the connecting edge (33 a, b) may have a curvature, typically with a curvature in point C, or an angle in point C, with one another side of point C a connecting edge portion (33a, and 33b), that are typically mirror images of one another in a longitudinal line (e.g. y-axis) through point C

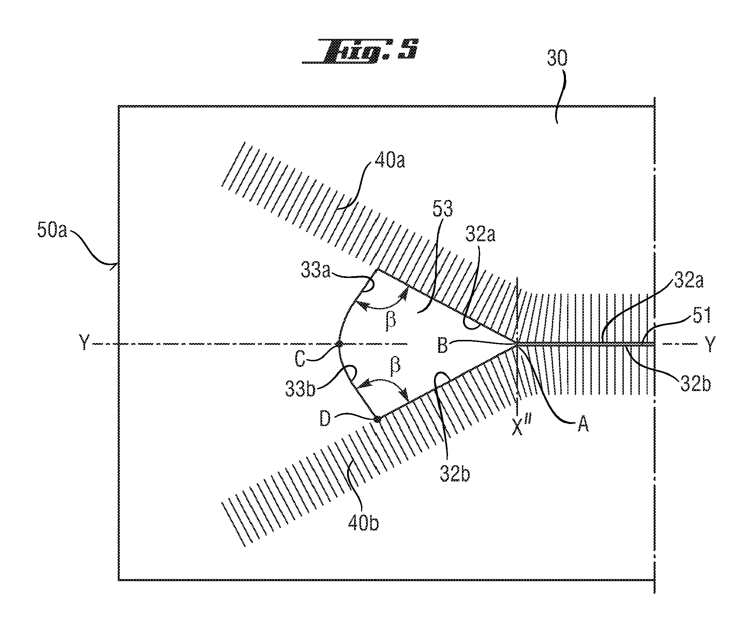

The elastic components (40) preferably are attached to the topsheet material with an angel or curvature in the back region (3), as for example shown in Figures 4 and 5. This helps to direct the elastic forces of the elastic components (40) to improve the alignment of the opening or openings with the anus (preferably and genitals) and to maintain the topsheet in contact or close proximity to the skin of the user. The elastic components (40) preferably have an angle or curvature with an angel or curvature point A. Preferably, opposing curvature or angel points A are positioned on the same transverse line.
Preferably at least one pair of elastic components is present such that they are mirror images of one another in the Y-axis and both curvature or angel points A are positioned on the same transverse line.
The transverse line through point A or points A is perpendicular to the Y-axis and crosses the Y-axis in point B. The distance between C and B is herein referred to as L".

In a preferred embodiment, the risk of substantive transverse wrinkles is reduced by limiting the ratio of length L to length L", i.e. to provide a topsheet (30) with connecting edge(s) (33), side edges (32) and elastic components (40) such that the ratio of L to L" is 0.55 or less, preferably 0.50 or less, and optionally at least 0.10 or at least 0.20.

The exact length of L and L" may depend on the total length of the topsheet, opening and elastic components, the required width of the opening and/ or the angle α.

Alternatively, or in addition, the topsheet (30) has in the back region (3) one or typically two (or more) triangular portions N, each defined (at 75% stretched state) by: i) a portion of the connecting edge (33 a or b) from point C to D (in said back region (3) of the topsheet (30)) and ii) at least part of the closest elastic component(s) (40 a or b) in said back region (3) of the topsheet (30); and iii) a line connecting i) and ii); whereby each such portion N (in 75% stretched state) is substantially free of transversely extending wrinkles, which means for the purpose of the invention that N has no transversely extending wrinkles of a height of more than 3 mm and/ or having an average transverse wrinkle height of less than 2 mm, as may be measured with PRIMOS equipment, and its data acquisition software, following the manufacture's instructions manual, using a 13x18mm lens (PRIMOS: using Light Source Schott KL1500, GFS Messtechnik GmbH, Germany).

Preferably, said portions N have no transversely extending wrinkles of a height of more than 2 mm, and if any transverse wrinkles are present, they have an average wrinkle height of less than 1 mm. More preferably, said portions N have no transversely extending wrinkles of a height of more than 0.5 mm. It may be preferred that portions N are free of any transversely extending wrinkles. (Portion N may have any size and triangular shape, provided the transverse wrinkles are as defined above.)

In a preferred embodiment, the absorbent article (1) of the invention has a topsheet (30) with one or more openings (31) and one or more elastic components (40 a, b) that comprises in the back region (3) a first portion (41 a, b) that is adjacent and substantially parallel and in close proximity to a closest (longitudinally extending) edge (32 a, b) of said opening (s) (31) (and optionally or typically said elastic component(s) (40 a, b) comprising in said back region (3) a third portion (43) that is not adjacent an edge of the opening (31) and whereby said elastic component(s) (40 a, b) comprises in said back region (3) of the topsheet (30) a second portion (42) that is adjacent and non-parallel to a closest edge (32, a, b) of said opening(s) (31), whereby this second portion (42) is minimised, i.e. whereby the length of said second portion (42) of the elastic component(s) (40 a, b) is less than L". In some embodiments, it may even be possibly to eliminate the presence of such a second portion (42), so that said length L is about 0, so that the angled or curved elastic components (40 a, b) comprise only said first and typically said third portion(s).

The topsheet (30) herein is the layer of the absorbent article (1) that in use is in contact or partial contact with the skin of the user. The topsheet (30) may be made of a single sheet or laminated sheet, or it may comprise separate sheets, that are connected or attached to one another, or it may comprise separate sheets that are configured such that they define said opening openings (31), described herein. In a preferred embodiment the topsheet (30) comprises at least a (optionally or preferably laminated) sheet that extends over about 70-100% of the width of the article (1) and about 70 to 100% of the length of the article (1).
In another preferred embodiment, the topsheet (30) may comprise two (optionally or preferably laminated) sheets that extend over about 70% to 100% of the length of the article (1), but only less than 50% of the width of the article (1), and which optionally may be directly attached to one another or connected to one another with an additional sheet, for example two longitudinally opposing substantially rectangular sheets in the XY plane (longitudinal x transverse direction plane), that may be mirror images of one another, and that may be connected to one another by a transversely position sheet, or strip in the X-Y plane or X-Z plane.

Such a single (optionally or preferably laminated) sheet or such sheets of the topsheet (30) in the back region (3) are preferably hydrophobic. Preferably, the topsheet (30) defines one or more opening (s) (31) along the longitudinal axis, which have a longitudinal dimension (length) substantially parallel to the longitudinal axis of the topsheet (30) and of the diaper. It may be preferred that the topsheet (30) defines one or two of such openings (31) or in a preferred embodiment the topsheet (30) has only one single opening (31).
It may be preferred that (in 100% stretched state) the opening (31) (or openings) of the topsheet (30) is (are) configured such that from 20% to 40%, or more preferably from 20% to 30% of the length of the opening (31) or total length of the opening(s) (31) extends from the transverse axis of the topsheet (30) towards the front edge of the topsheet (30), and the remaining percentage extends towards the back edge of the topsheet (30).
The dimensions and exact shape of the opening (31) may vary, depending on the size of the topsheet (30) and/ or the absorbent article (1).
In a preferred embodiment, the topsheet (30) defines one such opening (31) with a maximum length of from 40% to 90% or more preferably 50% to 80%, or even more preferably about 60% to 70%, of the total length of the absorbent article (1). The average width of the opening (31) herein may for example be from 5% to 30%, or more preferably 10% to 25%, of the average width of the absorbent articles (1) width along the transverse axis thereof.

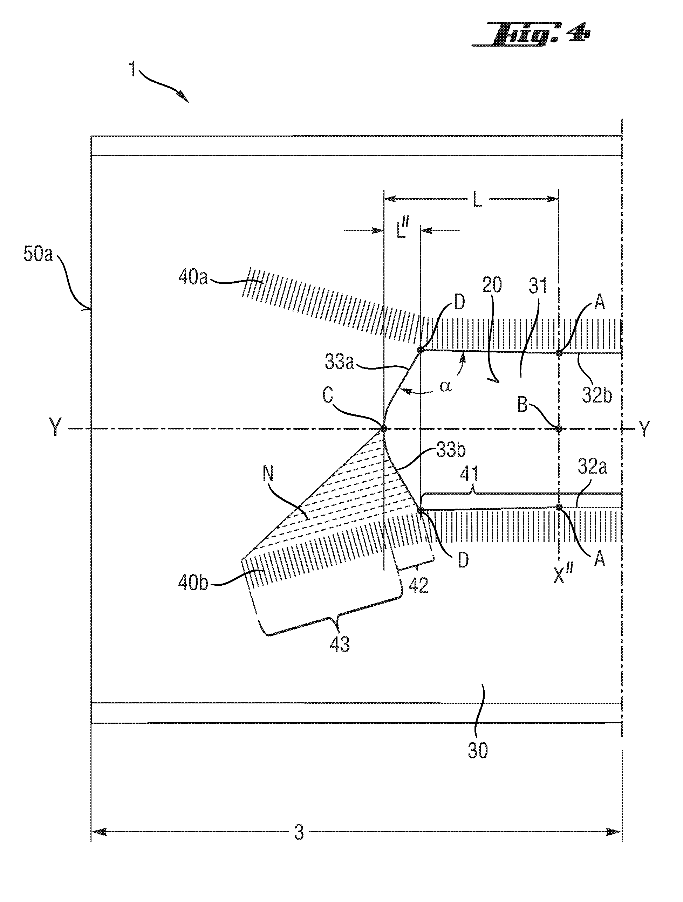

Preferably the opening(s) (31) has, in 100% stretched state, longitudinally extending opposing side edges (32 a, b) that are substantially parallel to the longitudinal axis of the article (1), as may be seen in Figure 3.
As mentioned above, the longitudinal side edges (32) are connected with one or more connecting edges (33 a, b) in at least the back region (3), typically one such connecting edge (33 a, b) in the back region, (but preferably also in the front region (2)), which may be a straight (preferably transverse) connecting edge, curved connecting edge or angled connecting edge, as can be seen from Figures 3 and 4.
In a preferred embodiment herein, said connecting edge (33 a, b) in the back region (3) of the topsheet (30) may be non-parallel but adjacent to part of a closest elastic component (40 a, b) over a length which is less than L", as can be seen in Figure 4.

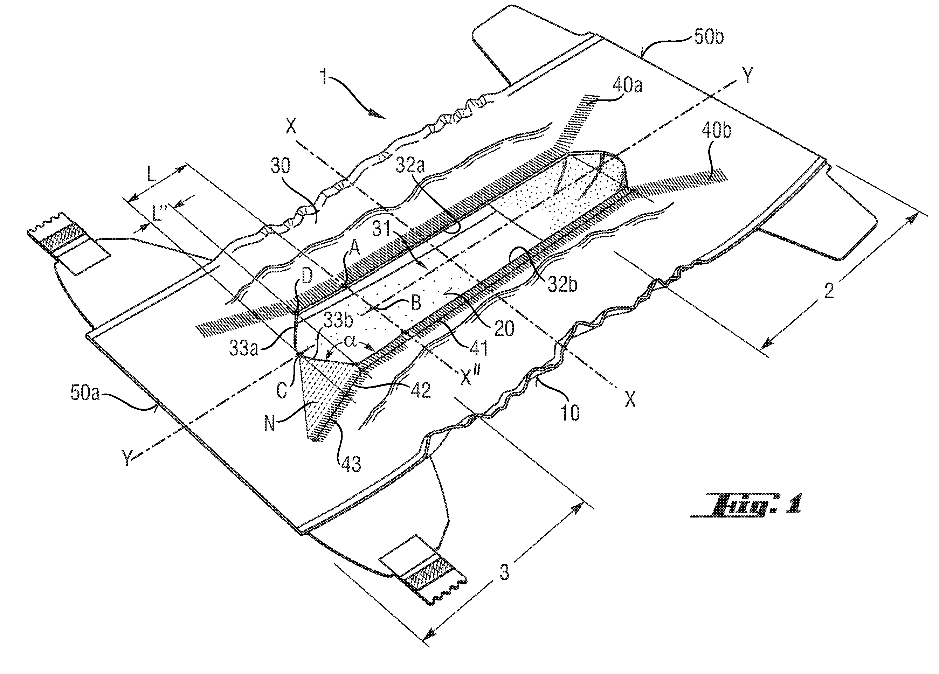

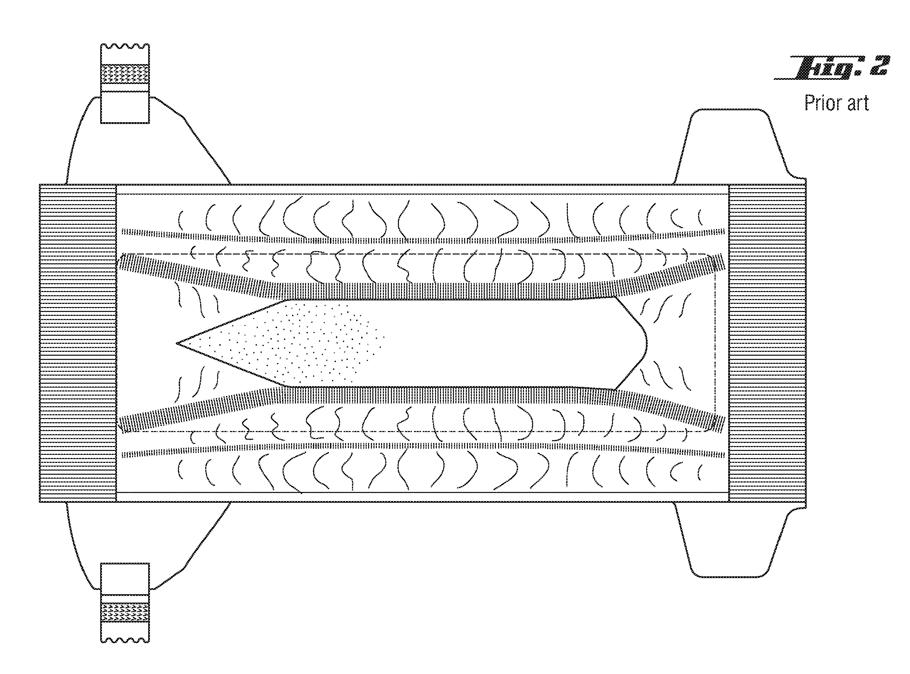

As described above, preferred articles herein comprise also one or more elastic components that (each) have a portion that is substantially parallel and in close proximity, i.e. within 3 mm, preferably within 2 mm, or even within 1mm, to the longitudinal extending edge (32 a, b) of an opening (31), as can be seen from Figures 1, 3 and 4.
The elastic component (40 a, b) extends typically (in longitudinal direction) beyond the opening (31) towards or to the transverse edge of the topsheet (30), as can be seen in the Figures, so that the elastic component (40 a, b) may have a third portion (43) that is not adjacent (an edge of) the opening (31).

The topsheet (30) herein comprises elastic components (40 a, b) that typically are attached with an angle or curvature in the back region (3), such that they at least in the back region (3) provide an angle or curvature. Preferably the article comprises a topsheet with at least a pair of opposing elastic components (40 a, b) that have a curvature or angle so they bend away from one another and away fromt eh Y-axis, towards the longitudinal edges (32 a, b) of the article (1).

The preferred elastic components (40) are diverting from the Y-axis. They are preferably attached such that: they have an angle (in point A; between the diverting elastic portion and a longitudinal line) of between 15° to 35° , preferably from 20° to 30°, or preferably from 24° and 28°; or that thay have a curvature with a curvature point A with an angle (between that the tangent line of the diverting elastic and a longitudinal line in said point A) of 15° to 35° , preferably from 30° to 20° preferably from 28° and 24°.

Preferably, the topsheet (30) comprises at least two elastic components (40 a, b) that are mirror images of one another (mirroring in the longitudinal axis).

The topsheet may also comprise one pair of elastic components (40 a, b) that have a portion in close proximity to the longitudinal edge of the opening in the back region (3), and in addition one or more elastic components positioned in the topsheet, but at a distance of at least 10 mm, preferably at least 15 mm or even at least 20 mm away from the closest longitudinal edge (40), so called secondary elastic components.

The elastic components herein (40 a, b) may for example be formed from (a multitude of thin) strands of elastic material or for example from a single band of elastic material.
The elastic components(s) extend in the back region (3) of the topsheet (30) beyond the longitudinal end edge or point of the opening (31) towards or to the transverse edge of the article (1). Typically, the length of each such elastic component (40 a, b) is preferably more than the length of the opening (31) or total length of the opening (31).
The width of the elastic components (40) on the topsheet (30) will vary, typically depending on the exact dimensions of the topsheet (30) and/ or the article (1). For example, the elastic component (40 a, b) may be from 0.5 mm to 40 mm, preferably 1 mm to 30 mm, or even more preferably 2 or 3 mm, or even 5 mm to 20 mm.

In one embodiment, the elastic topsheet herein has a specific force profile as described in co-pending European application No 01117670.8, and as determined by the method set out therein. In one embodiment, the elastic topsheet of the article has an elastic profile, based on a two-cycle hysteresis, measured by the method set out in the co-pending application mentioned above, using a 500mm/min clamp speed, which is as follows:

1.5Lt by a first load force of less than 1.1N, 3.0Lt by a first load force of less than 2.1N and 4.5Lt by a first load force of less than 3.0N and a second unload force at 4.5Lt of more than 0.9N, a second unload force at 3.0Lt of more than 0.5 and a second unload force at 1.5Lt of more than 0.1N.

More preferably, the profile of the elastic topsheet useful herein is:
1.5Lt by a first load force of less than 0.6 N, 3.0Lt by a first load force of less than 1.1N and 4.5Lt by a first load force of less than 1.5N and a second unload force at 4.5Lt of more than 0.9N, a second unload force at 3.0Lt of more than 0.5N and a second unload force at 1.5Lt of more than 0.1N.

Preferred profiles of a preferred elastic topsheet herein are defined by the first load force and second load forces at all of the lengths in the following table (for a two cycle hysteresis with 500 mm/ min clamp speed, stretching as set out below, up to 4.5Lt or 0.8Lₛ, which ever is smaller):

| | Preferred profile | More preferred profile | Most preferred profile |
|---|---|---|---|
| 1^{st} load force at 1.5Lt | < 1.1 N | < 1.1 N | < 0.6 N |
| 1^{st} load force at 2.0Lt | < 1.5 N | < 1.5 N | < 0.8 N |
| 1^{st} load force at 2.5Lt * | <1.8 N | <1.8 N | <0.9 N |
| 1^{st} load force at 3.0Lt * | < 2.1 N | < 2.1 N | < 1.1 N |
| 1^{st} load force at 3.5Lt * | <2.3 N | <2.3 N | <1.2 N |
| 1^{st} load force at 4.0Lt * | <2.6 N | <2.6 N | <1.3 N |
| 1^{st} load force at 4.5Lt * | <3.0 N | <3.0 N | <1.5 N |
| 2^{nd} unload force > at 1.5Lt | 0.1 N | > 0.2 N | > 0.1 N |
| 2^{nd} unload force > at 2.Lt | 0.3 N | > 0.6 N | > 0.3 N |
| 2^{nd} unload force > at 2.5Lt * | 0.4 N | > 0.8 N | > 0.4 N |
| 2^{nd} unload force > 0.5 N at 3.0Lt * | | > 1.0 N | > 0.5 N |
| 2^{nd} unload force > at 3.5Lt * | 0.6 N | > 1.2 N | > 0.6 N |
| 2^{nd} unload force > at 4.0Lt * | 0.7 N | > 1.4 N | > 0.7 N |
| 2^{nd} unload force > 0.9 N at 4.5Lt * | | > 1.8 N | > 0.9 N |

| | | | |
|---|---|---|---|
| *These values are only relevant as long as they are below 0.8Lₜₛ, as mentioned below in the test method. | | | |

In one embodiment, the article has a L_{c} which is less than 0.5Lₛ of the article. Preferably, L_{c} of the article is less than 0.45Lₛ of the article, or even less than 0.4Lₛ, or even less than 0.35Lₛ, or even less than 0.3Lₛ.

The elastic materials are typically in the form of a multitude of strands or a single band with an average thickness (e.g. gauge) of at least 20 microns, more preferably at least 40 microns, or even at least 60 microns, typically up to about 300 microns, or even up to 200 microns or even up to 150 microns. Highly preferred materials have an average thickness of about 70 to 100 microns. Preferred elastic materials used hereto include VFE-CD, available from Tredegar, and L-86, L-89, or L-90, available from Fulflex (Limerick, Ireland).

In order to further reduce the transverse wrinkles and risk of leakage, the topsheet (30) may comprise in the back region (3) and/ or in the portion(s) N an additional material, e.g. coating, additional sheet, to render the topsheet (30) stiffer in the back region (3) or portion(s) N compared to the front region (2) of the topsheet (30). Alternatively or in addition, the topsheet (30) may comprise in the back region (3) or portion (s) N an additional elastic component (40 a, b), or preferably an elastic sheet. Alternatively or in addition the topsheet (30) may comprise in the back region (3) or the portions(s) N a body adhesive that reduces the occurrence of transverse wrinkles and reduces leakage.

The absorbent article may comprise a genital coversheet that may be part of the topsheet (30) and/or connected to the sheet material of the topsheet (30). In use, this genital coversheet forms a partial pocket around the genitals, to protect the genitals from soiling.
Preferably, the genital coversheet is present in only the front region (2) of the absorbent article above or (preferably) below the elasticated sheet of the topsheet (30). The genital coversheet is preferably not completely attached to said elasticated sheet of the topsheet, and more preferably at the most partially or preferably not attached to the absorbent core (20) underneath it. Preferably, the maximum length of the part of the genital coversheet that is present above, in or under the opening (31)(s) is 10% to 50% of the maximum length of the opening (31), preferred 10% to 30%, or more preferably 13% to 28% or even more preferably 17% to 27%. In other words, at the most 50% of the maximum length of the opening (31) is 'covered' by the longest part of the genital coversheet, but at least 10% of the maximum length of the opening (31) is covered by the longest part of the genital coversheet.

In a preferred embodiment, the genital coversheet is extendable, either longitudinally or transversely or both, so that the pressure of the genitals onto the genital coversheet causes the genital coversheet to extend and form a pocket around the genitals. The genital coversheet may be elastically extendable, or preferably, the genital coversheet may be non-elastically extendable, in longitudinal and/ or, more preferably, in transverse direction.
The genital coversheet of the absorbent articles herein is preferably urine permeable.

The longitudinal side edges of the topsheet (30) are preferably joined or attached to the longitudinal side edges of the backsheet, by any attachment means known in the art, to form longitudinal opposing attachment areas. In one preferred embodiment of the present invention, the topsheet (30) and the backsheet are attached directly to one another in some locations and optionally indirectly joined together in other locations.
Preferably, the average width of the topsheet (30), including the width of the opening (31), is larger than the average distance between the longitudinal attachment areas of the topsheet (30) to the backsheet, mentioned above. Also preferred is that the average width of the topsheet (30), including the width of the opening (31), is larger than the average width of the backsheet. The topsheet (30) may for example have one or more transverse and/ or, more preferably, longitudinal folds, which can unfold in use and allow sagging of the core (20) and backsheet, while the topsheet (30) remains in place.
The topsheet (30) herein may be liquid or urine pervious or impervious. It may be preferred that the topsheet (30) is liquid or urine pervious in one direction, but liquid or urine impervious in the opposite direction, e.g. that body fluids may penetrate through the topsheet (30) to the remaining part of the diaper, but that no or limited amounts of liquid (urine) can penetrate in reverse direction, towards the wearer's skin. However, in a highly preferred embodiment, the topsheet (30) or at least more than 50% of its surface area (that faces the wearer in use) is hydrophobic. Preferred is that the topsheet (30) is urine impermeable and faeces impermeable.

Preferred topsheets (30) herein are made of a sheet or sheet of hydrophobic material or a sheet, or sheet or sheets that are treated to be hydrophobic (in order to isolate the wearer's skin from liquids contained in remaining part of the diaper), with for example a hydrophobic surface coating. Preferred hydrophobic surface coatings are for example described in co-pending application US60/543,785, filed February 11, 2004. Preferably, the hydrophobic surface coating comprises one or more silicone polymers or fluorinated polymers.
A suitable topsheet (30) may be manufactured from a wide range of materials, including woven or non-woven webs of natural fibers (e.g., wood or cotton fibers) or synthetic fibers (e.g., polyester, polyethylene and/ or polypropylene fibers), or a combination of natural and synthetic fibers. If the topsheet (30) includes fibers, the fibers may be for example spun bond, carded, wet-laid, melt blown, hydro entangled, or otherwise processed as is known in the art. Highly preferred are webs comprising spunbond layers (S) and meltblown layer(s) (M), whereby the surfaces of the web are formed by spunbond layer(s). Preferred are such webs with a relatively high basis weight, for example more than 25gram/ m² (gsm), for example
34gsm SMMS (whereby 12gsm meltblown and 5gsm spunbond); 34gsm SMMS (whereby 10gsm meltblown and 7gsm spunbond); 30gsm SMMS (whereby 10gsm meltblown and 5 gsm spunbond); 30gsm SMMS (whereby 8gsm meltblown and 7 gsm spunbond); 34gsm SMS (whereby 20gsm meltblow and 7gsm spunbond), or for example webs comprising two layers of 17 gsm SMMS, described above.
Any portion of the topsheet (30) may be coated with a lotion or anti-friction powder, known in the art.

The absorbent article also comprises a, typically liquid impervious, backsheet (10), as known in the art. Suitable backsheet materials comprise typically breathable material, which permit vapors to escape from the diaper while still preventing exudates from passing through the backsheet. Suitable backsheet films include those manufactured by Tredegar Industries Inc. of Terre Haute, IN and sold under the trade names X15306, X10962 and X10964. The backsheet, or any portion thereof, may be elastically extendable in one or more directions.
The backsheet (10) may be attached or joined to the topsheet (30), the absorbent core, or any other element of the diaper by any attachment means known in the art.
The attachment means to attach the topsheet (30) and the backsheet may include for example pressure bonds or for example a uniform continuous layer of adhesive, a patterned layer of adhesive, or an array of separate lines, spirals, or spots of adhesive, such as disclosed in U.S.4,573,986. Adhesives that have been found to be satisfactory are manufactured by H. B. Fuller Company of St. Paul, Minnesota and marketed as HL-1620 and HL-1358-XZP. Alternatively, the attachment means may comprise heat bonds, pressure bonds, ultrasonic bonds, dynamic mechanical bonds, or any other suitable attachment means or combinations of these attachment means as are known in the art.
The absorbent core may comprise any absorbent material which is generally compressible, conformable, non-irritating to the wearer's skin, and capable of absorbing and retaining urine, such as comminuted wood pulp, creped cellulose wadding; melt blown polymers, including coform; chemically stiffened, modified or cross-linked cellulosic fibers; tissue, including tissue wraps and tissue laminates; absorbent foams; absorbent sponges; super absorbent polymers; absorbent gelling materials; or any other known absorbent material or combinations of materials; preferred may be absorbent cores which have an absorbent storage layer which comprises more than 80% by weight of the absorbent core content (e.g. excluding core wrap, or coversheets) of absorbent gelling material, and which comprises less than 5%, or which is preferably free of, absorbent cellulose fibers such as pulp.

The absorbent article may also include a sub-layer disposed between the topsheet (30) and the absorbent core, capable of accepting, and/ or immobilizing bodily exudates, typically fecal material. Suitable materials for use as the sub-layer may include large cell open foams, macroporous compression resistant non woven highlofts, large size particulate forms of open and closed cell foams (macro and/or microporous), highloft non-wovens, polyolefin, polystyrene, polyurethane foams or particles, structures comprising a multiplicity of vertically oriented, preferably looped, strands of fibers, or preferably apertured formed films, as described above with respect to the genital coversheet. (As used herein, the term "microporous" refers to materials that are capable of transporting fluids by capillary action, but having a mean pore size of more than 50 microns. The term "macroporous" refers to materials having pores too large to effect capillary transport of fluid, generally having pores greater than about 0.5 mm (mean) in diameter and more specifically, having pores greater than about 1.0 mm (mean) in diameter, but typically less than 10 mm or even less than 6 mm (mean).

The absorbent article herein is preferably a disposable adult or infant diaper or training pants/pull-up pants. Diapers or training pants of the invention may have one or more pairs of elasticated leg cuffs that provide improved containment of liquids and other body exudates. Leg cuffs may also be referred to as leg bands, side flaps, barrier cuffs, or elastic cuffs, as described in; U.S. 3,860,003; U.S 4,808,178 and 4,909; U.S. 4,695,278 and 4,795,454.

### Preferred processes to make a diaper of the invention

The absorbent article (1) and the topsheet (30) with one or more openings (31) and elastic components (40 a, b), as described herein, may be made by any method. For example, the topsheet (30) may comprise two or more sheets or components that are assembled such that they define one or more opening (31) therein between, or one or more parts of the topsheet (30) may be removed to provide or more opening (31), or one or more cuts may be made in the topsheet (30) to provide one or more opening (31), or a combination of such techniques.

A preferred process (an example of a resulting material thereof is shown in Figure 5), comprising the steps of:
a) obtaining a sheet material with a longitudinal direction (Y) and a transverse direction (X) a back region (3) and a front region (2) and transverse end edges (50 a, b);
b) forming a central longitudinal slit (51) (cut) through at least part of the back region (3) of the sheet material in longitudinal direction Y, but not through the transverse end edges (50 a, b) of the sheet material and forming a two-dimensional opening (53), e.g. cut-out, for example a circular, oval, preferably diamond-shaped opening (53), in the back region (3) of the sheet;
c) prior, simultaneous with or subsequent to steps b) attaching one ore more elastic components (40 a, b) in stretched state to said sheet material with an angle or curvature in at lest the back region (3) of said sheet, as described herein.

It may be preferred that the opening (53)/ cut-out is done such that it will comprise on its edges point A, D and C, and B, (B may coincide with A). The article made by this process, may have the L and L" and ratio's and the angle α as defined above.
If one opening (31) is present in the topsheet (30), it may be preferred that said slit (51) is cut in part of the front and back region (3) of the article (1).
As can be seen from Figure 5, the opening (53) or cut-out may be in the shape of a "diamond" including a "diamond" with non-identical edges, and/ or rounded angles.

The preferred size of the slit and cut-out (51) will depend on the size of the topsheet (30) and absorbent article (1) and the elastic components (40 a, b) used. For example for a Pampers size 3 diaper (as sold in Germany) a suitable topsheet as described herein may be added with a opening length in 100% stretched state of 280 to 290 mm, which in use may provide an opening with a total length between 210 and 130 mm, depending on the baby.

A longitudinally extending (slit) opening (31) may be formed (cut) in the topsheet (30) material first and then a pair of elastic components (40 a, b) (e.g. bands) are attached, in partially or completely stretched state, to the topsheet (30), so that a first portion (41) of each elastic component (40 a, b) is present adjacent and in close proximity of a closest longitudinal edge, or part thereof, of the opening (31). Alternatively, the elastic components (40 a, b) may be first attached to the topsheet (30) in partially or completely stretched state, extending longitudinally over the topsheet (30), and having preferably an angle or curvature in at least the back region (3) of the topsheet (30), where after a longitudinally extending (slit) opening (31) is formed (cut) through part of the topsheet (30) material and part of the elastic components (40 a, b), typically along the longitudinal axis of the topsheet (30) material and the elastic components (40 a, b).
Attachment of the elastic components may be done by pressure and/ or heat and/ or adhesive. Preferred adhesives for attaching the elastic components include H2031, available from ATO-Findley and HL-1620 available from H.B. Fuller (St Pauls, USA).
In a preferred execution, the slit/ cut in the topsheet (30) may be cut as a slit, whereby the slit opening (31) obtains its width due to the spreading force of the angled/ curved elastic components (40 a, b), or the slit opening (31) may be cut with a width dimension, for example up to 3.0 cm. Typically, the slit opening (31) is cut as a slit without width dimension, except for the additional cut-out (51) in the back region (3) and attached to the slit in said back region (3), and optionally also a cut-out in the front region (2), attached to the slit edge in said front region point of the slit opening (31), as can be seen in Figure 5.

If the elastic components (40 a, b) are present on the surface of the topsheet (30) which is in contact with the skin of the user, it may be preferred that an additional layer, e.g. nonwoven material, is placed on the elastic components (40 a, b), to avoid direct contact by the elastic areas with the skin.

In a preferred execution, the elastic component (40 a, b)/ band is attached in a partially stretched state to each longitudinal edge of the opening (31) of the nonwoven, whereby a centre part is attached in partially or fully stretched state and the end portions of such an elastic component (40 a, b) are attached in unstretched state.

## Claims

1. An absorbent article (1) having a backsheet (10), an absorbent core (20) and a topsheet (30) with at least one opening (31) to receive fecal material, each having a longitudinal direction and axis (Y), and a transverse direction and axis (X), said topsheet (30) being made from one or more sheet material(s), and having a front region (2) and a back region (3), whereby at least one opening (31) or part thereof is present in at least the back region (3) of the topsheet (30), whereby in 100% stretched state:
a) said topsheet comprising at least one elastic component (40 a, b), whereby each elastic component (40 a, b) comprises a longitudinally extending portion and optionally being attached to said topsheet with a curvature, with a curvature point A, or an angle with an angle point A, present in the back region (3) of the topsheet (30);
b) at least one of said openings (31) or part thereof (3) having two longitudinally extending opposing edges (32 a, b) and at least substantially parallel to and in close proximity to each of said longitudinal edges or part thereof, at least one of said longitudinally extending portions of said elastic component (40 a, b); and said at least one opening (31) having at least one connecting edge (33 a, b), connecting said opposing longitudinal edges (32 a, b) in the back region (3);
whereby said connecting edge (33, a, b) connects to said longitudinal side edge in a point D (32 a, b) with angle α which is from 80° to 105°.

2. Process for making an absorbent article (1) having a backsheet (10), an absorbent core (20) and a topsheet (30) with at least one opening (31) to receive fecal material, each having a longitudinal direction and axis (Y), and a transverse direction and axis (X), said topsheet (30) being made from one or more sheet material(s), and having a front region (2) and a back region (3), whereby at least one opening (31) or part thereof is present in at least the back region (3) of the topsheet (30),
comprising the steps of;
a) obtaining a topsheet material with a longitudinal direction (Y) and a transverse direction (X) a back region (3) and a front region (2) and transverse end edges (50 a, b);
b) forming a central longitudinal slit (51) with slit edges through at least part of the back region (3) of the topsheet material in longitudinal direction Y, but not through the transverse end edges (50a, b) of the sheet material and forming a two-dimensional opening (53) in the back region (3) of the sheet, said opening (53) comprising a front connecting edge (33 a, b), and side edges that are connected to one another; and
c) prior, simultaneous with or subsequent to steps b) attaching one or more elastic components (40 a, b) in partially or completely stretched state to said sheet material with each an angle or curvature, having an angle or curvature point A, in at least the back region (3) of said topsheet material, and said elastic components (40 a, b) each comprising a longitudinally extending portion that is substantially parallel to and in close proximity to one of the longitudinal edges or part thereof of said slit (51);
whereby the angle α (in point D) between said front connecting edge (33 a, b) and a side edge of said opening (53) is from 80° to 105°.

3. An absorbent article obtainable by a process as in claim 2.

4. An absorbent article (1) as in claim 1 or 3 whereby each elastic component (40 a, b) is attached to said topsheet with a curvature, with a curvature point A, or an angle with an angle point A, present in the back region (3) of the topsheet (30); and whereby said connecting edge (33 a, b) in the back region (3) has a centre point C positioned on the longitudinal axis Y of said topsheet (30), and said connecting edge (33 a, b) or part thereof having a length L from point C to point D, and said topsheet (30) or topsheet material having one or more, preferably one, transverse line X" through said angle or curvature point A, and crossing the longitudinal axis Y perpendicularly in a point B, and having a length L" from a point B to point C, and whereby the ratio of L to L" is 0.55 or less, preferably 0.5 or less.

5. An absorbent article (1) as in claim 1 or 3 whereby said angle α is from 90° to 100°.

6. An absorbent article (1) as in any of the preceding claims 1 or 3 to 4 whereby the topsheet (30) has one longitudinally extending opening (31) with substantially parallel longitudinal side edges (32, a, b), present in the part of the front region (2) and part of the back region (3) of the topsheet (30).

7. An absorbent article (1) as in claim 4 whereby said elastic components (40 a, b) are in the form of at least two longitudinally extending elastic bands or strands, that are partially parallel to one another, and comprise each said curvature or angle in the back region (3), to divert the elastic components (40 a, b) and bend the elastic components (40 a, b) each away from the Y-axis.

8. Process as in claim 2 whereby said opening (53) is a diamond-shaped opening.

9. A process as in claim 2 whereby said elastic components (40 a, b) are in the form of at least two longitudinally extending elastic bands or strands, that are partially parallel to one another, and that are each attached to the topsheet with a curvature or angle with a said curvature or angle point A in at least the back region (3), to divert the elastic components (40 a, b) and bend the elastic components (40 a, b) each away from the y-axis, whereby each said elastic component (40 a, b) has an angle in said point A with the Y-axis or with a longitudinal line parallel to the Y-axis of from 15° to 35°, preferably from 20° to 30°.

10. An absorbent article (1) having a backsheet (10), an absorbent core (20) and a topsheet (30) with at least one opening (31) to receive fecal material, said topsheet (30) having a front region (2) and a back region (3), and having a longitudinal direction and axis (Y) and a transverse direction and axis X, the article (1) comprises a void space between said absorbent core (20) and said topsheet (30), whereby in 75% stretched state :
a) the topsheet (30) is made from one ore more sheet material(s) and comprises at least one elastic component (40 a, b),
b) each elastic component (40 a, b) is at least present along at least part of the longitudinal side edges (32 a, b) of the opening(s) (31), each elastic component (40 a, b) being attached to said topsheet with an angle or curvature, having an angle or curvature point A, in at least said back region (3), and each extending with an extending portion (43) towards the transverse edge and towards the longitudinal edge of the topsheet (30);
d) said opening (31) having a connecting edge (33 a, b), connecting the longitudinal edges (32 a, b) in said back region (3) in points D and each edge having a centre point C;
c) said topsheet (30) having in the back region (3) one ore more triangular portions N, each defined by: i) a portion of the connecting edge (33 a or b) from point D to C; ii) the closest elastic component extending portion (43), closest to said portion from point C to D; and iii) a line connecting i) and ii);
d) said portion(s) N being substantially free of transversely extending wrinkles, having no transversely extending wrinkles of a height of more than 3 mm and any transverse wrinkles present having an average transverse wrinkle height of less than 2 mm.

11. An absorbent article (1) as in claim 10, whereby each of said portion N has no transversely extending wrinkles of a height of more than 2 mm, and any transverse wrinkles present having an average wrinkle height of less than 1 mm.

12. An absorbent article (1) as in claim 10, whereby each of said portions N has no transversely extending wrinkles of a height of more than 0.5 mm, preferably whereby said portion(s) N is (are) free of any transversely extending wrinkles

13. An absorbent article (1) as in any of claim 1, 3 or 10 whereby the back portion (3) or part thereof of the topsheet (30) is stiffened, preferably by addition of additional sheet material in part of the back region (3).

14. An absorbent article (1) as in any of claims 1, 3 or 10 whereby the back portion (3) or part thereof of the topsheet (30) comprises an elastic sheet with preferably substantially transverse elasticity.

15. An absorbent article (1) as in any of claims 1, 3 or 10 whereby the back portion (3) or part thereof of the topsheet (30) comprises a body adhesive.
